# EUROPÄISCHE PATENTANMELDUNG

(11) **EP 1 523 962 A1**
(43) Veröffentlichungstag der Anmeldung: **20.04.2005**
(21) Anmeldenummer: 03023808.3
(22) Anmeldetag: 17.10.2003
(51) Int. Cl.: A61F 2/44

(54) **Satz von zervikalen Zwischenwirbelprothesen**

(71) Anmelder: Cervitech, Inc., Rockaway, NJ 07866 (US)
(72) Erfinder: Link, Helmut D., 22397 Hamburg (DE)
(74) Vertreter: Glawe, Delfs, Moll & Partner

(57) **Zusammenfassung**

Satz von zervikalen Zwischenwirbelprothesen, die ein Gelenk mit einem vorbestimmten Zentrum (18) der Gelenkbewegung haben. Zur besseren Anpassung an die unterschiedlichen Gelenkradien der zervikalen Bandscheiben ist vorgesehen, daß der Satz von zervikalen Zwischenwirbelprothesen mindestens zwei unterschiedliche Prothesen mit unterschiedlichem Gelenkradius umfaßt.

## Beschreibung

Die Erfindung bezieht sich auf zervikale Zwischenwirbelprothesen, die ein vorbestimmtes Zentrum der Gelenkbewegung haben. Bei einem ersten bekannten Typ solcher Prothesen befindet sich das Zentrum der Gelenkbewegung innerhalb der Prothese (US-A-5425773; EP-A-1166725). Dies stimmt mit den natürlichen Verhältnissen, die von der Prothese nachgebildet werden sollen, nicht überein. Bei einem anderem Typ von Zwischenwirbelprothesen (FR-A-2718635) wird das Gelenk von einem Gleitflächenpaar gebildet, dessen gemeinsames Krümmungszentrum außerhalb der Prothese, nämlich unterhalb derselben, liegt. Dies nähert sich den natürlichen Verhältnissen an, aber ist noch zu weit von ihnen entfernt.

Der Erfindung liegt die Erkenntnis zugrunde, daß die Zentren der zervikalen Zwischengelenkbewegung unterschiedlich sind von Wirbel zu Wirbel (L. Penning: Functional Pathology of the Cervical Spine; Excerpta Medica 1968, Seiten 1 - 23). Ausgehend von dieser Erkenntnis sucht die Erfindung eine stärkere Annäherung der prothetischen Gelenkbewegung an die natürlichen Verhältnisse.

Dies Ziel erreicht die Erfindung dadurch, daß sie einen Satz von zervikalen Zwischenwirbelprothesen zur Verfügung stellt, der mindestens zwei unterschiedliche Prothesen mit unterschiedliche Lage des Zentrums der Gelenkbewegung umfaßt. Je nach Lage des Falles kann der operierende Arzt durch Auswahl einer geeigneten Prothese aus diesem Satz dafür sorgen, daß die Bewegung des prothetisch versorgten Zwischenwirbelgelenks den natürlichen Verhältnissen ähnlicher ist, als dies bislang möglich war.

Das gilt insbesondere dann, wenn die Zwischenwirbelprothesen ein Gleitflächenpaar zur Bildung des Gelenks enthalten. In diesem Fall unterscheiden sich die beiden unterschiedlichen Prothesen durch unterschiedliche Krümmungsradien ihrer Gleitflächenpaare. Eine Prothese, die für ein mehr kranial liegendes Wirbelpaar bestimmt ist, soll erfindungsgemäß einen größeren Krümmungsradius ihrer Gleitflächen haben als eine Prothese, die für ein mehr kaudal liegendes Wirbelpaar bestimmt ist.

In manchen Fällen kann es genügen, wenn der Prothesensatz lediglich zwei Prothesen mit unterschiedlichem Krümmungsradius seiner Gleitflächen umfaßt, nämlich eine Prothese mit einem oberhalb und eine Prothese mit einem unterhalb eines bestimmten Zwischenwerts liegenden Krümmungsradius ihrer Gleitflächen. Dieser Zwischenwert liegt zweckmäßigerweise bei 18 mm. Beispielsweise kann ein Satz eine erste Prothese mit einem Krümmungsradius ihrer Gleitflächen von 22 mm und eine andere Prothese mit einem Krümmungsradius von 14 mm ihrer Gleitflächen umfassen. Erstrebenswert ist eine größere Anzahl von Prothesen mit unterschiedlichem Gleitflächenradius, beispielsweise kann der in dem soeben angegebenen Beispiel angegebene Satz ergänzt sein durch eine Prothese mit einem Gleitflächenradius von 18 mm und gegebenenfalls einer weiteren Prothese mit einem Gleitflächenradius von 10 mm.

Die Erfindung betrifft auch ein Verfahren zum Bestimmen der für einen zervikalen Bandscheibenersatz geeigneten Zwischenwirbelprothese aus einer Mehrzahl von Zwischenwirbelprothesen mit unterschiedlichem Gelenkradius. Es zeichnet sich dadurch aus, daß der Gelenkradius des betroffenen Gelenks bestimmt und eine Prothese mit einem diesem Gelenkradius nahekommenden Gelenkradius ausgewählt wird. Unter dem Gelenkradius ist in diesem Zusammenhang der Abstand zwischen dem Zentrum der Gelenkbewegung und dem Mittelpunkt der Prothese zu verstehen. Das Verfahren kann vom Arzt ausgeübt werden. In der Regel wird dieser jedoch die Bewegungscharakteristik des zu ersetzenden Gelenks wegen der vorhandenen Schäden nicht mehr feststellen können. Er wird deshalb darauf angewiesen sein, daß der Hersteller der Prothesen nach Durchführung geeigneter Ermittlungen, deren Ergebnis er auch der Literatur entnehmen kann, die von ihm zur Verfügung gestellten, satzweise zusammengestellten Prothesen bestimmten Wirbelzwischenräumen zuordnet. In der folgender Tabelle ist ein Beispiel für die Zuordnung der Krümmungsradien der Gleitflächen zu den einzelnen Zwischenwirbelräumen innerhalb bestimmter Größenreihen (in Millimeter) angegeben.

| **Zwischenwir- belraum** | **Reihe "groß"** | **Reihe "mittel"** | **Reihe "klein"** |
|---|---|---|---|
| C2/C3 | 22 | 20 | 18 |
| C3/C4 | 22 | 18 | 18 |
| C4/C5 | 18 | 18 | 16 |
| C5/C6 | 18 | 14 | 14 |
| C6/C7 | 14 | 14 | 12 |

Zu der Verwendung kleiner Krümmungsradien sei angemerkt, daß auch dabei das Gelenkzentrum außerhalb der Prothese liegt.

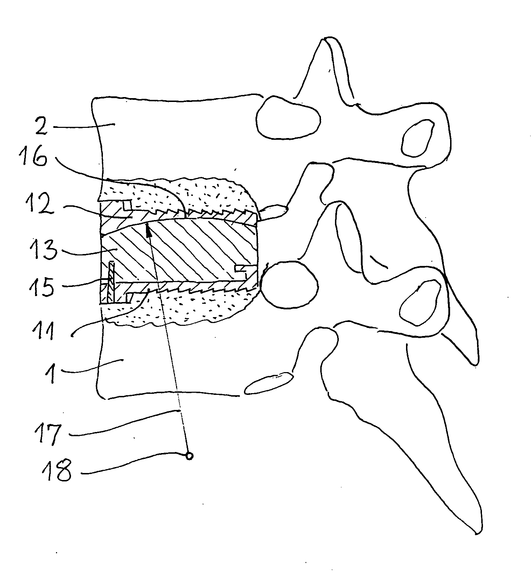

Die Zeichnung zeigt ein Ausführungsbeispiel zur Erläuterung der oben verwendeten Begriffe.

Zwischen die Wirbelkörper 1 und 2 ist eine Zwischenwirbelprothese eingesetzt, die aus einer unteren Deckplatte 11 einer oberen Deckplatte 12 und einem Prothesenkern 13 besteht. Dieser wird durch eine an drei Seiten der Prothese umlaufende, hinterschnittene Randleiste 14 und einen Riegel 15 an der unteren Deckplatte 11 festgehalten. Mit der oberen Deckplatte 12 bildet er ein sphärisches Gleitflächenpaar 16, das einen Gleitflächenradius 17 und einen Krümmungsmittelpunkt 18 aufweist, der das Bewegungszentrum des von der Prothese gebildeten Gelenks bildet. Das heißt, daß die Deckplatten 11, 12 und die mit ihnen verbundenen Wirbel 1, 2 eine Relativbewegung zueinander durchführen können, die sich als Rotationsbewegung um das Zentrum 18 darstellt, sofern das Gleitflächenpaar 16 alleine die Relativbewegung bestimmt. In der Praxis sind auch andere Gleitflächen, nämlich die Facettengelenke, an der Bestimmung der Relativbewegung beteiligt, so daß die tatsächlich sich einstellende Relativbewegung ein wenig davon abweichen mag. Es ist jedoch verständlich, daß die Gelenkbewegung um so harmonischer ist und fortdauernde Beschwerden des Patienten um so unwahrscheinlicher sind, je stärker das Zentrum 18 in der von der Prothese bestimmten Gelenkbewegung mit dem natürlich gegebenen Bewegungszentrum übereinstimmt. Der Gelenkradius wird unabhängig vom Gleitflächenradius bestimmt und unterscheidet sich von diesem dadurch, daß er vom Zentrum 18 der Gelenkbewegung zum geometrischen Mittelpunkt der Prothese gemessen wird.

Die für die mehr kranialen Zwischenwirbelräume (insbesondere C2/C3 und C3/C4) bestimmten Prothesen zeichnen sich einerseits durch einen geringeren Gelenkradius aus als die Prothesen, die für die mehr kaudal gelegenen Zwischenwirbelräume (insbesondere C5/C6 und C6/C7) bestimmt sind. Andererseits können die mehr kranial einzusetzenden Prothesen eine geringere Flächenausdehnung insbesondere in der AP-Richtung (AP = anterior-posterior) haben als die mehr kaudal einzusetzenden Prothesen. Daher besteht ein weiteres Kennzeichen der Erfindung darin, daß der Satz von Zwischenwirbelprothesen mindestens eine erste Prothese umfaßt, deren Gelenkradius größer und deren flächige Ausdehnung (insbesondere in AP-Richtung) geringer sind als diejenigen einer zweiten Prothese.

## Patentansprüche

1. Satz von zervikalen Zwischenwirbelprothesen, die ein Gelenk mit einem vorbestimmten Zentrum (18) der Gelenkbewegung haben, **dadurch gekennzeichnet, daß** er mindestens zwei unterschiedliche Prothesen mit unterschiedlicher Lage des Zentrums (18) der Gelenkbewegung umfaßt.

2. Satz von zervikalen Zwischenwirbelprothesen nach Anspruch 1 **dadurch gekennzeichnet, daß** die Prothesen ein Gleitflächenpaar (16) zur Bildung des Gelenks enthalten und die mindestens zwei unterschiedlichen Prothesen unterschiedliche Krümmungsradien (17) an ihrem Gleitflächenpaar (16) aufweisen.

3. Satz von zervikalen Zwischenwirbelprothesen nach Anspruch 2 **dadurch gekennzeichnet, daß** eine für ein mehr kranial liegendes Wirbelpaar bestimmte Prothese einen größeren Krümmungsradius (17) ihres Gleitflächenpaars (16) als eine für ein mehr kaudal liegendes Wirbelpaar bestimmte Prothese aufweist.

4. Satz von zervikalen Zwischenwirbelprothesen nach Anspruch 2 **dadurch gekennzeichnet, daß** er mindestens eine Prothese mit einem oberhalb und mindestens eine Prothese mit einem unterhalb 18 mm liegenden Gleitflächenradius (17) umfaßt.

5. Satz von zervikalen Zwischenwirbelprothesen nach Anspruch 2 oder 4 **dadurch gekennzeichnet, daß** er mindestens eine Prothese mit einem Gleitflächenradius unterhalb 15 mm umfaßt.

6. Satz von zervikalen Zwischenwirbelprothesen nach Anspruch 2 **dadurch gekennzeichnet, daß** er mindestens eine erste Prothese umfaßt, die eine geringere Ausdehnung in AP-Richtung und einen größeren Gleitflächenradius als eine zweite Prothese aufweist.

7. Verfahren zum Bestimmen der für einen zervikalen Bandscheibenersatz geeigneten Zwischenwirbelprothese aus einer Mehrzahl von Zwischenwirbelprothesen mit unterschiedlichem Gelenkradius **dadurch gekennzeichnet, daß** der Gelenkradius des betroffenen Gelenks bestimmt und eine Prothese mit einem diesem Gelenkradius nahekommenden Gelenkradius ausgewählt wird.
